# EUROPEAN PATENT APPLICATION

(11) **EP 4 327 733 A1**
(43) Date of publication of application: **28.02.2024**
(21) Application number: 23189666.3
(22) Date of filing: 04.08.2023
(51) Int. Cl.: A61B 5/021, A61B 5/0215, A61B 5/00, A61M 5/168, A61M 5/172, A61M 25/00

(54) **LUMEN DESIGN WITHIN INTRAVENOUS TUBE TO TRANSMIT BLOOD PRESSURE WAVE FOR INVASIVE BLOOD PRESSURE MONITORING**

(30) Priority: 23.08.2022 US 202217821624
(71) Applicant: Honeywell International Inc., Charlotte, NC 28202 (US)
(72) Inventor: KISHORE, Kuna Venkat Satya Rama, Charlotte, 28202 (US)
(74) Representative: Haseltine Lake Kempner LLP

(57) **Abstract**

A system and apparatus for utilizing invasive techniques to determine the blood pressure of a patient, are provided. An example system may include a pressure sensor, an intravenous fluid supply bag, and a hollow needle configured to penetrate a blood vessel of a patient. An intravenous supply tube may fluidly connect the pressure sensor to the hollow needle. A lumen filled with an incompressible fluid may be disposed within the intravenous supply tube. The lumen may be coupled to the pressure sensor at one end and terminate in a flexible membrane at the other end. The flexible membrane may deform in response to a blood pressure wave transmitted from the blood vessel of the patient, and transmit the blood pressure wave through the incompressible fluid and to the pressure sensor. The pressure sensor may determine a blood pressure measurement based at least in part on the received blood pressure wave.

## Description

### TECHNOLOGICAL FIELD

Embodiments of the present disclosure relate generally to hemodynamic monitors, and more particularly, to hemodynamic monitors utilizing invasive techniques to measure an intravenous blood pressure wave.

### BACKGROUND

Applicant has identified many technical challenges and difficulties associated with measuring a patient's blood pressure using invasive blood pressure monitoring techniques. Through applied effort, ingenuity, and innovation, Applicant has solved problems related to monitoring the blood pressure of a patient through an intravenous cannula by developing solutions embodied in the present disclosure, which are described in detail below.

### BRIEF SUMMARY

Various embodiments are directed to an example apparatus and system for monitoring a patient's blood pressure using an intravenous cannula.

In accordance with some embodiments of the present disclosure, an example hemodynamic monitoring apparatus is provided. In some embodiments, the hemodynamic monitoring apparatus may comprise a pressure sensor comprising a pressure sensing element and a tube filled with an incompressible fluid and disposed within an intravenous supply tube that is in fluid communication with the pressure sensor. In some embodiments, the tube may comprise a first end coupled to the pressure sensing element and a second end comprising a flexible membrane. In some embodiments, the flexible membrane may deform in response to a force such that the force may be transmitted through the incompressible fluid to the pressure sensing element. Further, the pressure sensor may determine a blood pressure based at least in part on the force.

In some embodiments, the pressure sensor may determine a blood pressure while an intravenous fluid simultaneously flows from an intravenous fluid supply, through the intravenous supply tube, and into a blood vessel of a patient.

In some embodiments, the pressure sensing element may be isolated from the intravenous fluid.

In some embodiments, the flexible membrane may protrude from the second end of the tube forming a rounded surface positioned to contact a fluid within the intravenous supply tube.

In some embodiments, the tube may terminate prior to entering a blood vessel of a patient.

In some embodiments, the flexible membrane may be isolated from contact with bodily fluids of the patient.

In some embodiments, the flexible membrane may comprise a thin membrane of biocompatible polyvinyl chloride material.

In some embodiments, the intravenous fluid may substantially fill the intravenous supply tube, such that a blood pressure wave is transmitted from a bodily fluid of the patient to the intravenous fluid in the intravenous supply tube to interact with the flexible membrane.

In some embodiments, the incompressible fluid may create a continuous medium for a blood pressure wave to propagate from the second end of the tube to the pressure sensing element.

In some embodiments, the incompressible fluid may comprise a high-viscosity, incompressible silicone material.

In some embodiments, the tube may comprise an inner diameter between 0.4 and 0.6 millimeters and an outer diameter between 0.9 and 1.1 millimeters.

An example hemodynamic monitoring system is further provided. In some embodiments, the system may comprise a pressure sensor comprising a pressure sensing element, an intravenous fluid supply bag containing intravenous fluid, and the intravenous supply bag being in fluid communication with the pressure sensor by a first intravenous supply tube. In some embodiments, the system may further include a hollow needle configured to penetrate a blood vessel of a patient, a second intravenous supply tube providing fluid communication between the pressure sensor and the hollow needle, and a tube filled with an incompressible fluid and disposed within the second intravenous supply tube. In some embodiments, the tube may comprise a first end coupled to the pressure sensing element and a second end comprising a flexible membrane, wherein the flexible membrane deforms in response to a force such that the force is transmitted through the incompressible fluid to the pressure sensing element. Further, the pressure sensor may determine a blood pressure based at least in part on the force.

In some embodiments, the pressure sensing element may be isolated from the intravenous fluid.

In some embodiments, the flexible membrane may protrude from the second end of the tube forming a rounded surface positioned to contact a fluid within the second intravenous supply tube.

In some embodiments, the tube may terminate prior to entering the blood vessel of the patient.

In some embodiments, the flexible membrane may comprise a thin membrane of biocompatible polyvinyl chloride material.

In some embodiments, the intravenous fluid may substantially fill the second intravenous supply tube, such that a blood pressure wave may be transmitted from a bodily fluid of the patient to the intravenous fluid in the second intravenous supply tube to interact with the flexible membrane.

In some embodiments, the incompressible fluid may create a continuous medium for a blood pressure wave to propagate from the second end of the tube to the pressure sensing element.

In some embodiments, the incompressible fluid may comprise a high-viscosity, incompressible silicone material.

In some embodiments, the pressure sensor may determine a blood pressure while the intravenous fluid simultaneously flows from the intravenous fluid supply bag, through the first intravenous supply tube and the second intravenous supply tube, into the blood vessel of the patient.

### BRIEF DESCRIPTION OF THE DRAWINGS

Reference will now be made to the accompanying drawings. The components illustrated in the figures may or may not be present in certain embodiments described herein. Some embodiments may include fewer (or more) components than those shown in the figures in accordance with an example embodiment of the present disclosure.
FIG. 1 illustrates an overall view of an example hemodynamic monitoring system in accordance with an example embodiment of the present disclosure.
FIG. 2 illustrates an example pressure wave transmission lumen of a hemodynamic monitoring system in accordance with an example embodiment of the present disclosure.
FIG. 3A illustrates a longitudinal cross-sectional view of a pressure wave transmission lumen within an intravenous (IV) tube in accordance with an example embodiment of the present disclosure.
FIG. 3B depicts a transverse cross-section of an example pressure transmitting IV supply tube in accordance with an example embodiment of the present disclosure.
FIG. 4 illustrates an example cannula and pressure transmitting IV supply tube in accordance with an example embodiment of the present disclosure.
FIG. 5A illustrates a longitudinal cross-section view of an example pressure transmitting IV supply tube interfacing with a pressure transducer in accordance with an example embodiment of the present disclosure.
FIG. 5B illustrates a close-up view of an example pressure transmitting IV supply tube interfacing with a pressure transducer in accordance with an example embodiment of the present disclosure.
FIG. 6 illustrates an example blood pressure wave propagating through the hemodynamic monitoring system in accordance with an example embodiment of the present disclosure.

### DETAILED DESCRIPTION

Example embodiments will be described more fully hereinafter with reference to the accompanying drawings, in which some, but not all embodiments of the inventions of the disclosure are shown. Indeed, embodiments of the disclosure may be embodied in many different forms and should not be construed as limited to the embodiments set forth herein; rather, these embodiments are provided so that this disclosure will satisfy applicable legal requirements. Like numbers refer to like elements throughout.

Various example embodiments address technical problems associated with performing invasive hemodynamic measurements from an intravenous blood pressure wave. As understood by those of skill in the field to which the present disclosure pertains, there are numerous example scenarios in which a patient's hemodynamic characteristics, including blood pressure, may need to be measured using an invasive intravenous device.

The conventional non-invasive method of blood pressure measurement uses a constricting cuff and a listening device, such as a stethoscope to determine the blood pressure in a patient's blood vessels. Such methods involve inflating the cuff to block circulation through a patient's arteries. The pressure on the cuff is then slowly released. The medical professional then listens to the flow of blood through the patient's arteries and determines the point at which blood freely flows through the artery. This point can then be correlated to the pressure induced by the cuff to determine a patient's blood pressure. The conventional method can depend largely on the size and tightness of the cuff and is highly susceptible to human error. In addition, the conventional method only reflects the blood pressure at a single point in time. For these reasons, conventional blood pressure measurement devices are not used when the accuracy and immediacy of the blood pressure measurements are critically important, for example, in and after surgery and/or with critically ill patients.

Invasive measuring techniques have been developed to provide medical professionals with more accurate and real-time blood pressure measurements. Invasive blood pressure monitoring involves direct measurement of intravenous blood pressure by inserting a hollow needle apparatus, or cannula, into a blood vessel. The hollow needle may be connected to an intravenous (IV) supply tube filled with pressurized IV fluid or other fluid and further connected to a pressure transducer containing a media-isolated or similar pressure sensor. When a patient's heart beats, a blood pressure wave is generated and propagated through the patient's artery, through the cannula, and through the IV fluid to the pressure sensor of the pressure transducer. The pressure transducer may continuously monitor the change in pressure and output a pressure waveform associated with the patient's real-time blood pressure measurements.

However, common invasive blood pressure monitoring techniques may also be susceptible to inaccuracies. For example, a conventional invasive blood pressure monitoring system utilizes the fluid in the IV supply line to hydraulically couple the blood pressure from the patient's artery to the pressure transducer connected to the IV supply line. In order to accurately measure the patient's blood pressure, the pressure transducer must be positioned at the same height as the phlebostatic axis of the patient (anatomical point corresponding to the right atrium). Misalignment may lead to erroneous blood pressure readings. A pressure transducer positioned out of alignment by as little as 3 centimeters may result in a pressure error at or near 2 mmHg, which may be outside of the acceptable limit. The reasons for such blood pressure error may be from hydrostatic pressure acting on the IV fluid in the tube when the relative height between the pressure transducer and the phlebostatic axis changes. The additional hydrostatic pressure or reduction in hydrostatic pressure, may be measured as an actual blood pressure measurement that is being transmitted hydraulically through the IV fluid in fluid communication with the patient's blood vessel. Patient movements may cause such a change in relative height between the pressure transducer and the phlebostatic axis resulting in a change in hydrostatic pressure, leading to inaccurate blood pressure measurements. In some instances, the patient movement may lead to an increase in hydrostatic pressure, while in other instances the patient movement may lead to a decrease in hydrostatic pressure, both adversely affecting the blood pressure measurements. Further, some invasive blood pressure monitoring techniques insert a small tube or measuring device through a cannula and into a patient's body vessel to provide real-time blood pressure measurements while a patient is in the intensive care unit (ICU) or during a surgical procedure, such as angioplasty. These devices may require a patient to be unconscious under general anesthesia. Thus, such invasive devices may not be used to measure blood pressure during post-surgery recovery monitoring. Further, these devices interact directly with the bodily fluids of the patient, with various additional considerations and regulations.

The various example embodiments described herein utilize various techniques to ensure accurate readings when utilizing an invasive hemodynamic monitoring system. For example, in some embodiments, a tube, or lumen, is positioned within the IV supply line between the pressure transducer and the cannula providing IV fluid to the patient. In some embodiments, the lumen may be filled with an incompressible fluid or gel which interacts with the intravenous blood pressure wave and propagates the waveform from within the IV supply tube proximate the cannula to the pressure transducer. The incompressible fluid within the lumen is unaffected by changes in hydrostatic pressure, allowing accurate blood pressure wave propagation, even when the patient and/or pressure transducer are moved. In addition, in some embodiments, the end of the lumen proximate the cannula connector may include a flexible membrane or bulb. Such a flexible membrane may increase the surface area of the lumen interacting with the blood pressure wave and improve the blood pressure wave propagated to the pressure transducer. In addition, in some embodiments, the lumen of the hemodynamic monitoring system described herein may terminate before entering the cannula housing, and a body vessel of the patient. As such, real-time intravenous blood pressure measurements can be obtained while the patient is conscious. Such real-time intravenous blood pressure measurements enable medical professionals to closely monitor the real-time operation of the heart, as well as determine characteristics of the heart based on the detected blood pressure waveform.

As a result of the herein described example embodiments and in some examples, the accuracy and comfort of invasive hemodynamic measuring devices may be greatly improved. In addition, the accuracy of the hemodynamic measurements may not be susceptible to patient movements and instrument misalignments.

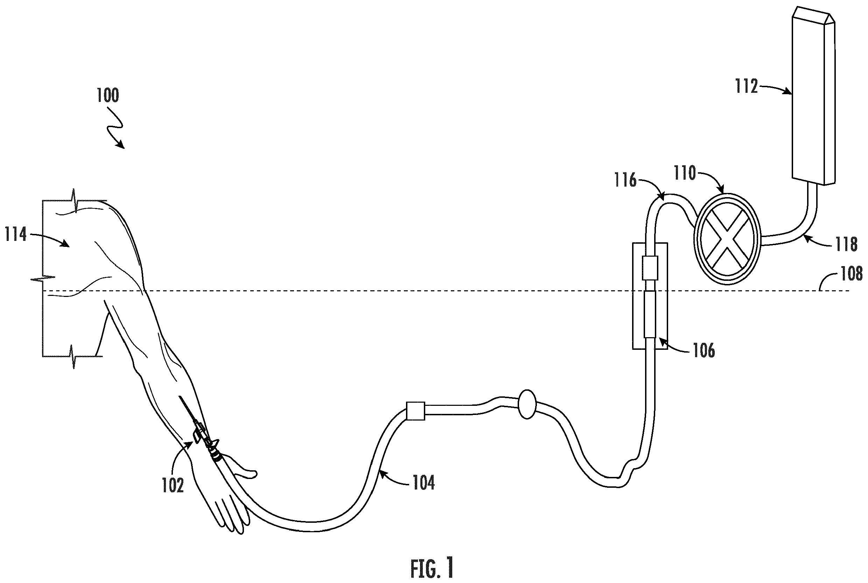

FIG. 1 illustrates an overview of an example hemodynamic monitoring system 100 according to one or more embodiments of the present invention. As depicted in the example embodiment of FIG. 1, the example hemodynamic monitoring system 100 includes an intravenous (IV) fluid supply bag 112 containing a pressurized supply of IV fluid. The IV fluid supply bag 112 is fluidly connected to roller clamp 110 via an IV supply tube 118. An IV supply tube 116 further fluidly connects the roller clamp 110 to the blood pressure transducer 106. The blood pressure transducer 106 is further fluidly connected to a cannula 102 via a pressure transmitting IV supply tube 104. The cannula 102 is further inserted to a blood vessel of the patient 114 to simultaneously provide IV fluid to the patient 114 and monitor the patient's 114 hemodynamic measurements.

As illustrated in FIG. 1, the example hemodynamic monitoring system 100 includes an IV fluid supply bag 112 containing IV fluid. An IV fluid supply bag 112 may be any bag, reservoir, container, receptacle, and/or the like capable of holding a supply of IV fluid or other similar fluid. In some embodiments, the IV fluid supply bag 112 may comprise vinyl or soft plastic. The IV fluid contained within the IV fluid supply bag 112 may be intended to enter the patient's 114 body through a cannula 102 inserted into a blood vessel of a patient 114. In some embodiments, the IV fluid supply bag 112 may be pressurized to overcome the pressure within the blood vessels of the patient 114 to allow the IV fluid to flow through the cannula 102 into the veins, and further to prevent blood within the blood vessels from exiting the blood vessels. An IV fluid supply bag 112 may be pressurized by elevating the bag, by applying a constricting sleeve or band around the IV fluid supply bag 112, or by a similar method. In some embodiments, the IV fluid supply bag 112 may be pressurized to about 300 mmHg and arranged to flow at a rate of about 5 milliliters per hour. The IV fluid supply bag 112 releases IV fluid into IV supply tube 118 which is fluidly connected to the roller clamp 110.

As further illustrated in FIG. 1, the example hemodynamic monitoring system 100 includes a roller clamp 110. A roller clamp 110 may be utilized by a medical professional to adjust the flow rate of IV fluid into a patient 114 utilizing a hemodynamic monitoring system 100. The roller clamp 110 may be continually adjusted to ensure the proper flow of IV fluid is introduced into the system of the patient 114. The roller clamp 110 may receive IV fluid from the IV fluid supply bag 112 and release IV fluid into the blood pressure transducer 106 via an IV supply tube 116.

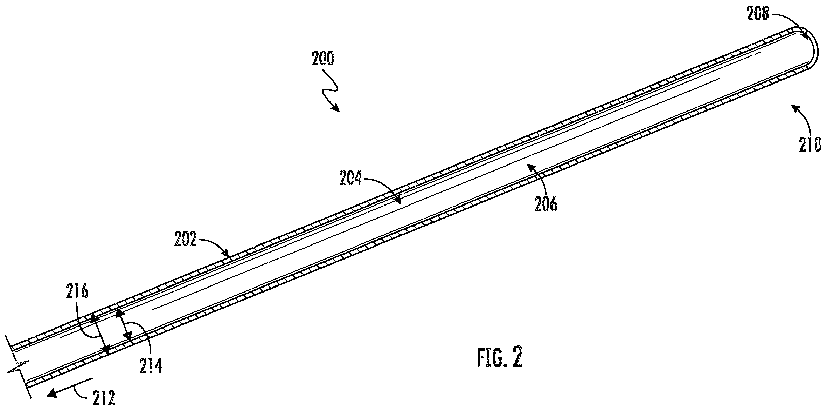

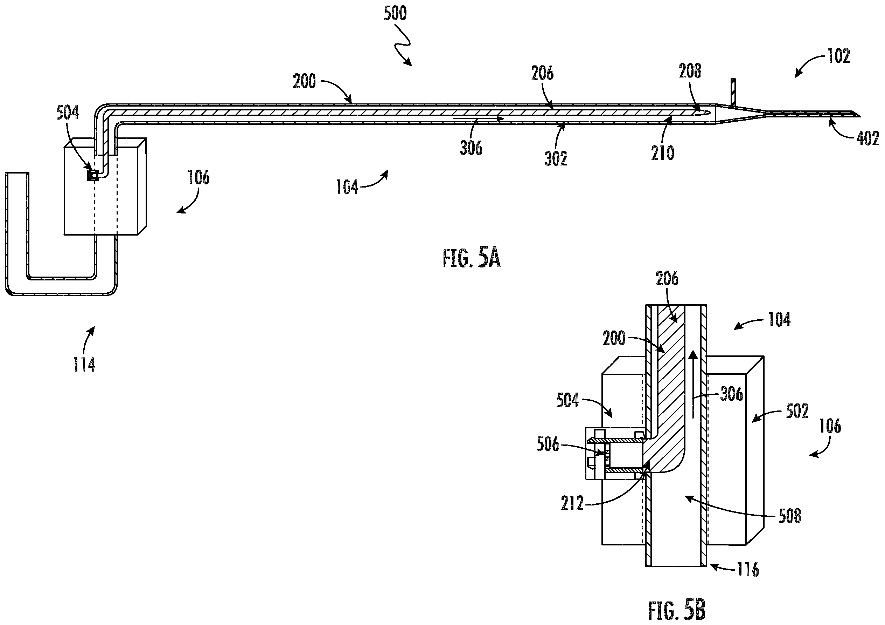

As further illustrated in FIG. 1, the blood pressure transducer 106 is fluidly connected to the roller clamp 110 and receives IV fluid from the roller clamp 110 through IV supply tube 116. Further, the blood pressure transducer 106 releases IV fluid to the cannula 102 through pressure transmitting IV supply tube 104. In addition, and as further described in FIG. 2 - FIG. 6, the blood pressure wave from within the patient's 114 body cavity, is propagated through the cannula 102 and through the pressure transmitting IV supply tube 104 to the blood pressure transducer 106. As further described in relation to FIG. 5A - FIG. 6, the blood pressure transducer 106 may contain components to determine and output a real-time pressure reading based on the blood pressure wave propagated through the pressure transmitting IV supply tube 104.

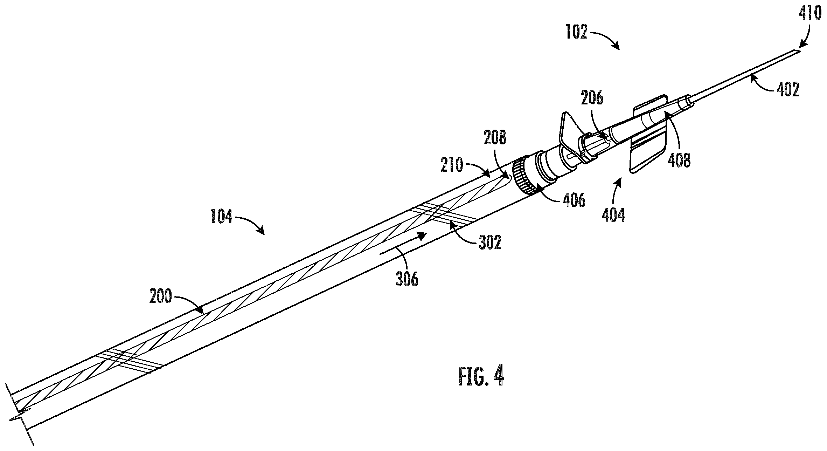

As further illustrated in FIG. 1, the cannula 102 receives IV fluid from the blood pressure transducer 106 via pressure transmitting IV supply tube 104. As described in relation to FIG. 4 - FIG. 6, the cannula 102 may be any device that provides access to the patient's 114 hemodynamic measurements through propagation of a blood pressure wave. As further described herein, the cannula 102 may release IV into a body cavity (e.g., blood vessel) of the patient 114 while simultaneously allowing transmission of the blood pressure wave through the cannula 102 and into the pressure transmitting IV supply tube 104.

Referring now to FIG. 2, a longitudinal cross-section of an example pressure wave transmission lumen 200 is provided. The depicted pressure wave transmission lumen 200 includes a lumen wall 202 enclosing a lumen conduit 204. FIG. 2 further shows the lumen conduit 204 filled with an incompressible fluid 206. Further, the pressure wave transmission lumen 200 includes a flexible membrane (e.g., bulb 208) proximate the distal end 210 of the lumen conduit 204.

As depicted in FIG. 2, the example pressure wave transmission lumen 200 includes a lumen wall 202 enclosing a lumen conduit 204. The example pressure wave transmission lumen 200 may be any structure that includes at least one sidewall (e.g., lumen wall 202) forming a perimeter around an interior opening (e.g., lumen conduit 204) and provides an enclosed, uninterrupted conduit along the length of the lumen. In some embodiments, the pressure wave transmission lumen 200 may have an inner diameter 214 between 0.4 and 0.6 millimeters; more preferably between 0.45 and 0.55 millimeters; most preferably between 0.48 and 0.52 millimeters. In addition, in some embodiments, the pressure wave transmission lumen 200 may have an outer diameter 216 between 0.8 and 1.2 millimeters; more preferably between 0.9 and 1.1 millimeters; most preferably between 0.95 and 1.05 millimeters. In some embodiments, lumen wall 202 may comprise a biocompatible material such as polyvinyl chloride, polyethylene, thermoplastic elastomers, polypropylene plastic, or other similar material. The lumen wall 202 may provide a sealed conduit, such that the fluid, gel, or other liquid contained in the lumen conduit 204 may not permeate the lumen wall 202. In some embodiments, the lumen wall 202 may be transparent, such that the lumen conduit 204, and the contents of the lumen conduit 204 may be visible.

As further depicted in FIG. 2, the example pressure wave transmission lumen 200 includes an incompressible fluid 206, substantially filling the lumen conduit 204. The incompressible fluid 206 may be any incompressible material capable of providing a coupling mechanism between the distal end 210 and the attached end 212 (as further shown in FIG. 5A) of the pressure wave transmission lumen 200. In some embodiments, the incompressible fluid 206 may comprise a high-viscosity fluid, such as silicone. In some embodiments, the pressure wave transmission lumen 200 may be pre-filled with the incompressible fluid 206 such that substantially all voids and air pockets are removed from the lumen conduit 204. As a coupling mechanism, any blood pressure wave interacting with the incompressible fluid 206 at the distal end 210 of the pressure wave transmission lumen 200 may be propagated through the lumen conduit 204 to the attached end 212 of the pressure wave transmission lumen 200. Since the incompressible fluid 206 in the lumen conduit 204 is not susceptible to hydrostatic pressure, the blood pressure waves received by the blood pressure transducer 106 are not adversely affected by patient 114 movement.

As further depicted in FIG. 2, the example pressure wave transmission lumen 200 includes a flexible membrane (e.g., bulb 208) at the distal end 210. The bulb 208 may be any flexible membrane that creates a fluid barrier between the incompressible fluid 206 in the lumen conduit 204 and the outside environment. In some embodiments, the bulb 208 may comprise a thin membrane of biocompatible material such as polyvinyl chloride. The bulb 208 protruding from the distal end 210 of the pressure wave transmission lumen 200 creates a greater surface area to interact with a blood pressure wave. The increased surface area and flexibility of the bulb 208 allow the bulb 208 to deform in response to a blood pressure waveform. Such deforming, or pressure coupling, enables the blood pressure waveform to propagate through the pressure wave transmission lumen 200, using the incompressible fluid 206 as the propagating medium.

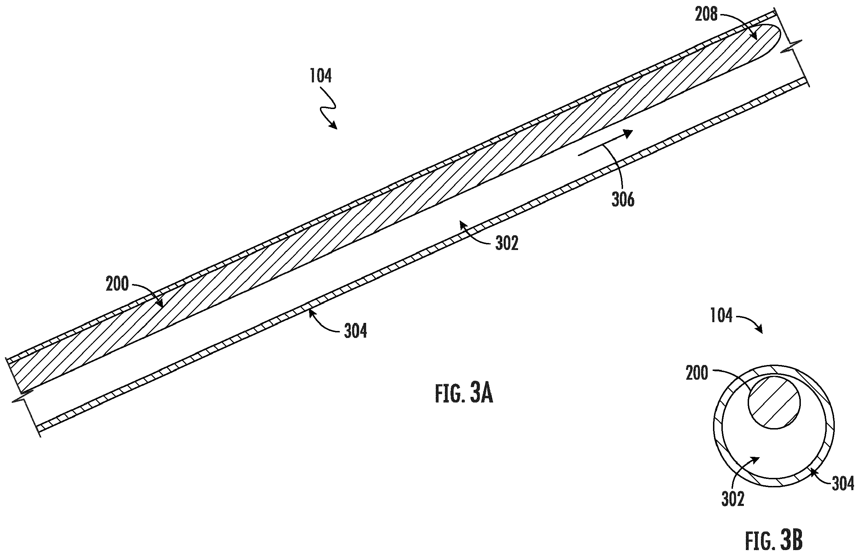

Referring now to FIG. 3A, a longitudinal cross-section of an example pressure transmitting IV supply tube 104 is provided. As depicted in FIG. 3A, the example pressure transmitting IV supply tube 104 includes an IV supply tube wall 304 forming a perimeter around an IV supply conduit 302. Within the IV supply conduit 302 a pressure wave transmission lumen 200 is disposed. In addition, the excess space within the IV supply conduit 302 is substantially filled with IV fluid 306 flowing toward the patient 114.

As illustrated in FIG. 3A, the example pressure transmitting IV supply tube 104 includes an IV supply tube wall 304 forming a perimeter around an IV supply conduit 302. In some embodiments, the IV supply tube wall 304 forming the perimeter of the pressure transmitting IV supply tube 104 may comprise a biocompatible material such as polyvinyl chloride, polyethylene, thermoplastic elastomers, polypropylene plastic, or other similar material. The IV supply tube wall 304 may provide a sealed conduit, such that the IV fluid 306, or other liquid contained in the IV supply conduit 302 may not permeate the IV supply tube wall 304. In some embodiments, the IV supply tube wall 304 may be transparent, such that the IV supply conduit 302, the pressure wave transmission lumen 200, and the contents of the pressure transmitting IV supply tube 104 may be visible.

As further illustrated in FIG. 3A, the example pressure transmitting IV supply tube 104 contains IV fluid 306 within the IV supply conduit 302 in the space unoccupied by the pressure wave transmission lumen 200. FIG. 3B further depicts a transverse cross-section of the pressure transmitting IV supply tube 104. As shown in FIG. 3B, the pressure wave transmission lumen 200 is positioned within the perimeter of the IV supply tube wall 304 such that the IV fluid 306 substantially fills the IV supply conduit 302. The IV fluid 306, thus, continues to flow in the space of the IV supply conduit 302 not occupied by the pressure wave transmission lumen 200 toward the patient 114.

Referring now to FIG. 4, an example pressure transmitting IV supply tube 104 is shown interfacing with an example cannula 102. As depicted in FIG. 4, the example cannula 102 includes a cannula housing 404 providing a leak-free connection to the pressure transmitting IV supply tube 104. The cannula further includes a hollow needle 402 in which the needle conduit 410 is fluidly connected to the IV supply conduit 302 through the leak-free connector 406 and the cannula conduit 408 of the cannula housing 404.

As illustrated in FIG. 4, the example hemodynamic monitoring system 100 includes a cannula 102. The cannula 102 may be any device or structure comprising a thin tube that may be inserted into a patient's 114 body cavity, for example, a blood vessel, to provide a fluid connection from the pressure transmitting IV supply tube 104 to the patient's 114 body cavity. The example cannula 102 further comprises a cannula housing 404. In some embodiments, the cannula housing 404 may comprise a body or structure to defining a conduit (e.g., cannula conduit 408) providing a fluid connection between the IV supply conduit 302 and the patient's 114 body cavity. The cannula housing 404 may further provide structures and/or features to support the hollow needle 402 and provide convenience in inserting the hollow needle 402 into the patient's 114 blood vessel or other cavity. In some embodiments (not shown) the cannula housing 404 may further include additional structures providing connection points for administering medications and drawing blood; structures for preventing back flow of bodily fluids; and/or the like.

As further depicted in FIG. 4, the example cannula 102 further includes a leak-free connector 406. The leak-free connector 406 may be any connecting structure that fluidly connects the IV supply conduit 302 to the cannula conduit 408 such that fluid leaks are substantially limited. In some embodiments, the leak-free connector 406 may comprise a Luer connector, Luer-lock fitting, Luer taper, or other similar leak free connection.

As further depicted in FIG. 4, the example cannula 102 further includes a hollow needle 402. The hollow needle 402 (e.g., catheter) may be any thin tube comprised of medical grade materials that may be inserted into a body cavity of a patient 114 (e.g., a blood vessel) and provide fluid communication, for example through a narrow conduit (e.g., needle conduit 410), to the body cavity of the patient 114 from outside the body of the patient 114. In some embodiments, the hollow needle 402 may further include a pointed tip to pierce the skin of the patient 114 and insert the end of the hollow needle 402 into the body cavity of the patient 114. In some embodiments, the hollow needle 402 may be attached to the cannula housing 404, such that the needle conduit 410 of the hollow needle 402 may be fluidly coupled with the cannula conduit 408, such that a fluid, for example IV fluid 306, may flow from the IV supply conduit 302, through the cannula conduit 408 and the needle conduit 410 and into the body cavity of the patient 114.

As further depicted in FIG. 4, the cannula 102 may be attached to the pressure transmitting IV supply tube 104. As described in relation to FIG. 3A, a pressure transmitting IV supply tube 104 may comprise a pressure wave transmission lumen 200 disposed within the IV supply conduit 302. The distal end 210 of the pressure wave transmission lumen 200 may terminate in a flexible membrane (e.g., bulb 208). In some embodiments, the bulb 208 may be proximate the interface of the cannula housing 404 and the pressure transmitting IV supply tube 104. In some embodiments, the bulb 208 may be within the cannula housing 404, for example, in the cannula conduit 408. Such close proximity to the body cavity of the patient 114 allows the pressure wave transmission lumen 200 to accurately propagate the blood pressure wave from the patient's 114 body to the blood pressure transducer 106.

Referring now to FIG. 5A, an example hemodynamic monitoring system 500, (e.g., pressure monitoring system utilizing catheters and tubes) is provided. The depicted hemodynamic monitoring system 500 of FIG. 5A includes pressure transmitting IV supply tube 104 comprising an IV supply conduit 302 and a pressure wave transmission lumen 200 disposed within the IV supply conduit 302. The pressure transmitting IV supply tube 104 is attached to a cannula 102 at a first end and a blood pressure transducer 106 at a second end, providing a fluid connection through which IV fluid 306 may flow from the blood pressure transducer 106 to the cannula 102. Additionally, the blood pressure transducer 106 is fluidly connected to a fluid source, such as an IV fluid supply bag 112 as described in FIG. 1, by IV supply tube 116.

FIG. 5B provides a detailed view of the example blood pressure transducer 106. As depicted in FIG. 5B, the example blood pressure transducer 106 includes a pressure sensor 504 comprising a pressure sensing element 506 and fluidly connected to the attached end 212 of the pressure wave transmission lumen 200. FIG. 5B further depicts a pressure transducer conduit 508 within the transducer housing 502 fluidly coupling the IV supply tube 116 to the pressure transmitting IV supply tube 104.

As illustrated in FIG. 5B, the example blood pressure transducer 106 includes a transducer housing 502. The transducer housing 502 may be any structure capable of accommodating a pressure sensor 504 and providing a conduit, such as pressure transducer conduit 508 through which IV fluid 306 may flow. In some embodiments, the transducer housing 502 may further provide leak-free connections, such as a Luer connector, to attach each of the IV supply tube 116 and the pressure transmitting IV supply tube 104 to the pressure transducer conduit 508. The transducer housing 502 may further provide structures and/or features, to support and/or attach a pressure sensor 504. The structures and/or features of the transducer housing 502 may position the pressure sensor 504, such that the pressure sensing mechanism is accessible from within the pressure transducer conduit 508.

As further illustrated in FIG. 5B, the example blood pressure transducer 106 includes a pressure sensor 504. A pressure sensor 504 may be any electrical, mechanical, and/or electromechanical device capable of generating a signal as a function of the pressure imposed by the surrounding atmosphere. In some embodiments, the pressure sensor 504 may comprise a gel-coupled pressure sensor. In such an embodiment, a gel or other material may be used to receive the pressure inducing forces and transmit the pressure forces to the pressure sensing element 506. As described, the pressure sensor may further comprise a pressure sensing element 506. In some embodiments, the pressure sensing element 506 may comprise a semiconductor material. In some embodiments, the pressure sensing element 506 may contain structures that change properties based on the force applied to the surface of the pressure sensing element 506. For example, the pressure sensing element may comprise piezoresistive sensors arranged in a Wheatstone bridge circuit, such that pressure on and deflection of the pressure sensing element 506 creates a change in resistance in the disposed sensors and a voltage is output correlated to the force applied to the surface of the pressure sensing element from the surrounding environment. In some embodiments, the pressure sensing element 506 may be a component of a media-isolated pressure sensor. In a media-isolated pressure sensor, the pressure sensing element 506 is isolated from the measured media. In such an embodiment, a coupling mechanism may be placed on top of the pressure sensing element 506 to interact with the surrounding environment and determine a pressure reading.

As further depicted in FIG. 5B, an opening of the pressure sensor 504, from which the pressure of the surrounding environment is measured, is coupled with the pressure wave transmission lumen 200, such that a blood pressure wave that interacts with the bulb 208 at the distal end 210 of the pressure wave transmission lumen 200 is propagated through the incompressible fluid 206 and directly to the pressure sensor 504.

In prior art examples, a pressure sensor would interact with IV fluid, such that, a blood pressure wave may travel through a cannula and through an IV supply conduit utilizing the IV fluid as a transmission medium, and interact with the pressure sensor directly. Because of this, pressure measurements using this technique were susceptible to inaccuracies due to patient movement. In these prior art examples, a pressure sensor was required to be precisely placed at the phlebostatic axis of the patient's chest. Patient movements, relative to the phlebostatic axis may cause changes in hydrostatic pressure within the IV supply tube, causing changes in the blood pressure measurement.

By attaching the pressure wave transmission lumen 200 directly to the pressure sensor 504, the pressure sensor 504 may be unaffected by patient 114 movements. In some embodiments, the blood pressure wave may pass into the cannula conduit 408 and interact with the bulb 208 at the distal end 210 of the pressure wave transmission lumen 200. The blood pressure wave may further propagate through the incompressible fluid 206 of the pressure wave transmission lumen 200 and interact with the pressure sensing element 506 of the pressure sensor 504. The fluctuations in pressure may be detected by the pressure sensor 504 and output as a blood pressure waveform or in another format representative of the pressure measured.

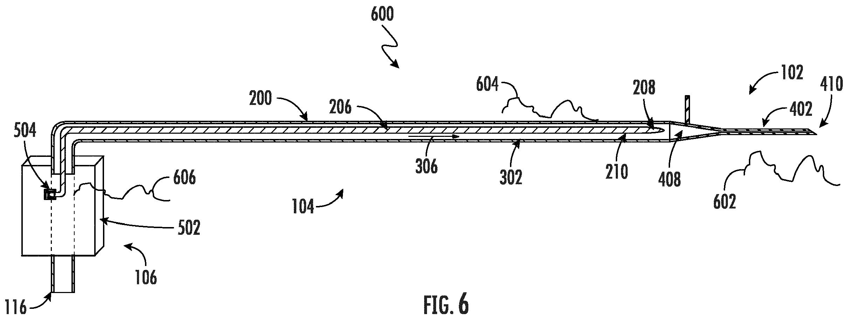

Referring now to FIG. 6, an example hemodynamic monitoring system 600 is provided. The example hemodynamic monitoring system 600 of FIG. 6 depicts a patient blood pressure wave 602 as it may exist exiting the body cavity of the patient 114 and entering the needle conduit 410 of the hollow needle 402. Each of the blood pressure waves (e.g., patient blood pressure wave 602, lumen blood pressure wave 604, sensor blood pressure wave 606) depict an example blood pressure measurement (for example in mmHg) with respect to time.

In some embodiments, the blood pressure wave may continue through the cannula conduit 408 where the blood pressure wave interacts with the flexible membrane (e.g., bulb 208) of the pressure wave transmission lumen 200. The blood pressure wave interacting with the bulb 208 is subsequently propagated through the incompressible fluid 206. The lumen blood pressure wave 604 depicts the blood pressure wave as it may exist as it propagates through the incompressible fluid 206. As compared to the patient blood pressure wave 602 existing at the point of entry into the cannula 102, the magnitude of the lumen blood pressure wave 604 may be reduced due to losses in the propagation and transmission to the incompressible fluid 206.

In some embodiments, the blood pressure wave may continue through the pressure wave transmission lumen 200 until the blood pressure wave arrives at the pressure sensor 504 coupled to the pressure wave transmission lumen 200. The waveform depicted as sensor blood pressure wave 606 represents the blood pressure wave as it may exist when it is received at the pressure sensor 504. The magnitude of the blood pressure waveform as depicted in sensor blood pressure wave 606 has been further reduced as compared to lumen blood pressure wave 604 due, once again, to losses during propagation. However, the pressure sensor 504 may determine the original blood pressure wave as it occurred within the body cavity of the patient 114. In some embodiments, the pressure transmitting IV supply tube 104 may have a pre-determined length and subsequently, the pressure wave transmission lumen 200 may also have a pre-determined length. With a known length of propagation in the incompressible fluid 206, the amount of loss due to propagation through the incompressible fluid 206 may be determined. Utilizing this information, the blood pressure waveform as it existed before propagating through the incompressible fluid 206 may be determined.

Many modifications and other embodiments of the inventions set forth herein will come to mind to one skilled in the art to which these inventions pertain having the benefit of teachings presented in the foregoing descriptions and the associated drawings. Although the figures only show certain components of the apparatus and systems described herein, it is understood that various other components may be used in conjunction with the system. Therefore, it is to be understood that the inventions are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

While various embodiments in accordance with the principles disclosed herein have been shown and described above, modifications thereof may be made by one skilled in the art without departing from the spirit and the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure.

Use of broader terms such as "comprises," "includes," and "having" should be understood to provide support for narrower terms such as "consisting of," "consisting essentially of," and "comprised substantially of' Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

## Claims

1. An apparatus comprising:
a pressure sensor comprising a pressure sensing element; and
a tube filled with an incompressible fluid and disposed within an intravenous supply tube that is in fluid communication with the pressure sensor, the tube comprising:
a first end coupled to the pressure sensing element; and
a second end comprising a flexible membrane;
wherein the flexible membrane deforms in response to a force such that the force is transmitted through the incompressible fluid to the pressure sensing element, and
wherein the pressure sensor determines a blood pressure based at least in part on the force.

2. The apparatus of Claim 1, wherein the pressure sensor determines a blood pressure while an intravenous fluid simultaneously flows from an intravenous fluid supply, through the intravenous supply tube, and into a blood vessel of a patient.

3. The apparatus of Claim 1, wherein the flexible membrane protrudes from the second end of the tube forming a rounded surface positioned to contact a fluid within the intravenous supply tube.

4. The apparatus of Claim 2, wherein the tube terminates prior to entering the blood vessel of the patient.

5. The apparatus of Claim 2, wherein the flexible membrane is isolated from contact with bodily fluids of the patient.

6. The apparatus of Claim 1, wherein the incompressible fluid creates a continuous medium for a blood pressure wave to propagate from the second end of the tube to the pressure sensing element.

7. The apparatus of Claim 1, wherein the incompressible fluid comprises a high-viscosity, incompressible silicone material.

8. A hemodynamic monitoring system, comprising:
a pressure sensor comprising a pressure sensing element;
an intravenous fluid supply bag containing intravenous fluid, the intravenous supply bag being in fluid communication with the pressure sensor by a first intravenous supply tube;
a hollow needle configured to penetrate a blood vessel of a patient;
a second intravenous supply tube providing fluid communication between the pressure sensor and the hollow needle; and
a tube filled with an incompressible fluid and disposed within the second intravenous supply tube, the tube comprising:
a first end coupled to the pressure sensing element; and
a second end comprising a flexible membrane;
wherein the flexible membrane deforms in response to a force such that the force is transmitted through the incompressible fluid to the pressure sensing element, and
wherein the pressure sensor determines a blood pressure based at least in part on the force.

9. The hemodynamic monitoring system of Claim 8, wherein the pressure sensing element is isolated from the intravenous fluid.

10. The hemodynamic monitoring system of Claim 8, wherein the flexible membrane protrudes from the second end of the tube forming a rounded surface positioned to contact a fluid within the second intravenous supply tube.

11. The hemodynamic monitoring system of Claim 8, wherein the tube terminates prior to entering the blood vessel of the patient.

12. The hemodynamic monitoring system of Claim 8, wherein the flexible membrane comprises a thin membrane of biocompatible polyvinyl chloride material.

13. The hemodynamic monitoring system of Claim 8, wherein the incompressible fluid creates a continuous medium for a blood pressure wave to propagate from the second end of the tube to the pressure sensing element.

14. The hemodynamic monitoring system of Claim 8, wherein the incompressible fluid comprises a high-viscosity, incompressible silicone material.

15. The hemodynamic monitoring system of Claim 8, wherein the pressure sensor determines a blood pressure while the intravenous fluid simultaneously flows from the intravenous fluid supply bag, through the first intravenous supply tube and the second intravenous supply tube, into the blood vessel of the patient.
